# EUROPEAN PATENT APPLICATION

(11) **EP 0 790 052 A1**
(43) Date of publication of application: **20.08.1997**
(21) Application number: 97102237.1
(22) Date of filing: 12.02.1997
(51) Int. Cl.: A61K 7/00, A61K 7/13, A61K 7/032

(54) **Method and compound to colour the hair at the root**

(30) Priority: 19.02.1996 IT PC960003
(71) Applicant: STEP S.r.l., 29015 Castelsangiovanni (PC) (IT)
(72) Inventor: Ghignone, Irma, 29015 Castelsangiovanni (PC) (IT)
(74) Representative: La Ciura, Salvatore

(57) **Abstract**

This invention relates to a method and a relevant compound to colour the hair from root.

According to the invention a compound is prepared, which can be either liquid or in slabs, which is based on mascara.

This compound shall be applied by means of a brush, thus colouring the root of the hair without resorting to a new dyeing.

## Description

The following invention relates to a method and a relevant compound to colour the hair at the base, and particularly refers to a system that allows, using a brush, to colour the base of the hair to cover the hair growth, that is to colour the part of the hair which, following a dyeing, appear to be white after the hair's growth.

The invention relates to the use of a compound based on ciliary encaustics, known as the English word "mascara".

According to the invention a compound is prepared, which can be either liquid or in slabs, that shall be applied by means of a brush, thus colouring the base of the hair without resorting to a new dyeing, with a simple operation and by using harmless products and therefore can be used at will.

As it is known, one of the problems that arises for people who resort to the hair dyeing is that few days after the treatment, because of the hair growth, at the root of the hair one can see an unaesthetic white part.

For this reason some products to colour the hair root have been developed, but these are products that solve only partially the problem.

These known products are made of stick dyeing, obtained by mixing a colouring pigment with a solid or half-solid carrier.

These products have the inconvenience to make the skin and the clothes greasy and, if not of good quality, they can drip causing the inconveniences that are deriving.

Furthermore the know stick products are often influenced by the light so it may happen that several products, a few hours after the application, change their colour, giving thus other problems.

The aim of this invention is proposing a method and a compound that allows to colour the hair near the root but that does not give the problems of the known products.

For this purpose, the invention provides for the use of products based on mascara, that are applied by a brush.

It is then enough dipping the brush in the product, giving a quick coat of paint on the hair's root and brushing the hair, to obtain the effect without the difficulties of the stick-products.

Other compounds based on mascara are already known, but they are used only for the cosmetic treatment of the eyes, in particular for the make up of the eyelash.

They are encaustic, or inks in which the coloured elements are mixed with a coating agent of different kinds, depending on the different types of encaustic.

They must have a considerable covering effect and for this reason the pigments are used in strong doses, usually not less then 10%.

The invention relates to use of these encaustic to dye the root of the hair and the preparation of the compounds can be obtained following technologies known in the field..

For instance, the compounds to dye the hair at the root could be prepared both in solid or liquid form.

In the case of solid compounds, these would liberate a fluid emulsion once they get in touch with a wet brush.

The hue, in several colours, can be obtained from an anhydrous cream based on bees-wax or carnauba wax and greasy carrier (for example lanolin with non-ionic and anionic emulsifying agents and even a coating agent like shellac).

In the case of liquid encaustic it is necessary to prepare a dispersion that does not need adding of water when applied.

In this case one can prepare a carrier from a stearic cream with a low content of saturated and fluid grease, whose part of water is made dense by polyvinyl alcohol and plasticized acetyl-vinyl resins.

In this carrier the pigments of the right colour are scattered, to obtain a liquid encaustic that can be applied by a brush.

If the encaustic is liquid, the product must be shaken before using to uniformly disperse the mineral charge.

This phenomenon can be limited by adding little percentages of not anionic surfactant agents to the compound, to delay the sedimentation.

The liquid compound can be prepared in a warm solvent under stirring, by hydrating the coating agents.

The pigments are then added and hold in suspension by shaking when the carrier is homogeneous.

The compound should preferable be water-repellent, to avoid inconveniences in case the hair becomes caught in the rain etc...

As it will be apparent from the above description, the use of the compound according to the invention is very simple.

It is enough dipping the brush in the compound, after having moistened it in the case one uses a solid compound, and rub it on the hair, near the root.

The base of the hair is thus coloured and it is enough, at the end of the treatment, to brush the hair to remove a possible surplus of compound.

This last, as said, can be applied without problems, since it is completely harmless and it is not irritant at all.

The product is cheap to be produced and easy to be applied and can be produced in the variety of colour requested.

An expert of this field could foresee several changes and variations, that must be included within the range of the present invention.

## Claims

1. Use of the mascara for the preparation of a compound to colour the hair at the root.

2. Method for colouring the hair at the root, characterised by the fact that a colouring compound, based on mascara, is applied to the base of the hair.

3. Method for colouring the hair at the root according to claim 1, characterised by the fact that said colouring compound is applied by means of a brush.

4. Compound to colour the hair at the root, characterised by the fact of comprising a dyeing based on mascara.

5. Compound to colour the hair at the root according to claim 4, characterised by the fact that it is obtained from an anhydrous cream based on bees-wax or carnauba wax and a greasy carrier.

6. Compound according to claim 5, characterised by the fact that the carrier said carrier is lanolin with non-ionic or anionic emulsifying agents.

7. Compound to colour the hair at the root according to claim 4, characterised by the fact that it is prepared from a stearic cream with a low content of saturated and fluid grease whose part of water is made dense by polyvinyl alcohol and plasticized acetyl-vinyl resins.

8. Compound according to claims 4 to 6, wherein said compound is in a solid state and is picked up by a wet brush.

9. Compound according to claim 7 wherein said compound is in a liquid state.
